# EUROPEAN PATENT APPLICATION

(11) **EP 3 578 143 A1**
(43) Date of publication of application: **11.12.2019**
(21) Application number: 17888796.4
(22) Date of filing: 14.04.2017
(51) Int. Cl.: A61F 2/95

(54) **DEVICE AND METHOD FOR SAFELY POSITIONING A CORONARY STENT IN THE CORONARY ARTERIES**

(30) Priority: 30.12.2016 RU 2016152734
(71) Applicant: Seven Sons Ltd. R.N. 515985570 (The "Company"), 67443 Tel Aviv (IL)
(72) Inventor: SEMISYNOV, Iliay Vladimirovich, Moscow 121165 (RU)
(74) Representative: Loven, Keith James
(86) International application number: PCT/RU2017/000235
(87) International publication number: WO 2018/124924

(57) **Abstract**

The present invention is suitable for use in medicine to enable a maximally accurate, fast and safe positioning of a coronary stent in case of uncomplicated and complicated anatomic lesions of the coronary bed as well as for stenting renal, visceral arteries. The claimed device comprises the housing with the tail, the screw rotating wheel, screw, runner with thread elements, scale for identification of the runner with the elastic coupling with placed elastic fixing elements, the plug consisting of 2 elements, the spring and the fixing button placed on the housing. In the preparation for use, a coronary stent carried by the delivery system is in advance progressively inserted inside the device following which the coronary stent carried by the delivery system is manually advanced to the affected area of the coronary artery, the delivery system is fixed and the stent is positioned in the coronary artery with the screw rotating wheel. The second embodiment example comprises the housing with the tail, the screw rotating wheel, the screw, the rotation gear, the hold-down cylinder, the fixing button with springs for placement of the guides for the delivery system inside the housing. In the preparation for use, a coronary stent carried by the delivery system is in advance progressively inserted inside the device following which the coronary stent carried by the delivery system is manually advanced to the affected area of the coronary artery, the delivery system is fixed and the stent is positioned in the coronary artery with the screw rotating wheel.

## Description

The present invention is intended for use in medicine in order to enable a maximally accurate, fast and safe positioning of a coronary stent in case of uncomplicated and complicated anatomic lesions of the coronary bed, in particular within the coronary artery in case of endovascular surgery by means of coronary stenting for recanalization of the artery portions constricted as a result of lesion.

The device is intended for use in case of coronary ostial lesions, coronary bifurcation lesions, including lesions of the left coronary artery trunk and ostial lesions of the right coronary artery, as well as in case of "stent-to-stent" implantation for reducing the "overlap" area.

The use of this device allows the radiation exposure of patients and medical personnel as well as the exposure of patients to radio-opaque contrast agents to be significantly reduced.

The device may be successfully used by young professionals at the time of their professional development in the field of interventional cardiology.

In addition, the claimed device may be also used for stenting renal, visceral arteries in a similar way.

A number of devices for positioning a stent within the coronary artery are available at the medical equipment market.

In particular, the Patent No. US 6293964 discloses OSTIAL PRO available from Merit Medica (USA). The device according to this patent comprises a cone made up of four tongues insertable into a guiding catheter together with a coronary stent. The guiding catheter is selectively positioned within the coronary ostium. The coronary stent is guided along the catheter to a location more distal that the coronary ostium, whereafter the guiding catheter is withdrawn from the coronary ostium and the device is deployed. As the device leaves the guiding catheter, its tongues unfold and abut against the aortal wall so that the guiding catheter tip cannot be selectively positioned within the coronary ostium as required for ostial stenting of the left coronary artery trunk and the right coronary artery trunk. The entire subsequent positioning of the coronary stent within the affected area is performed manually.

A disadvantage of the known device is its limited applicability, in particular only in cases of ostial lesions of the left coronary artery trunk and the right coronary artery trunk, as well as the manual positioning of the coronary stent.

Also known is a device for a safe positioning of a coronary stent within coronary arteries according to the Patent of the Russian Federation No. 2598798 owned by Seven Sons OJSC.

The claimed device differs primarily in:
- faster and more accurate positioning of a coronary stent within coronary artery,
- mechanical positioning rather than manual positioning of the stent,
- reduced radiation exposure of patients and medical personnel due to reduced time of the coronary stent positioning,
- applicability of the device in case of lesions within any segment of the coronary bed,
- reduced exposure of patients to radio-opaque contrast agents due to reduced time of the coronary stent positioning,
- single applicability,
- the device is conveniently configured as extension of the surgeon's arm,
- minimal weight and dimensional parameters,
- ease of use,
- cost-effective production.

The device is industrially applicable.

The claimed device comprises the housing with the tail and different elements placed inside the housing: the screw rotating wheel and the screw that are made as a single element, runner with the arrow and thread elements for making contact with the screw, scale for identification of the runner distance, coupling with placed elastic fixing elements, the plug consisting of 2 elements, the spring and the fixing button.

The cylindrical housing is made from plastic using the casting method under pressure, being pebbled at spots, where the operator touches the device using his/her arm, with the right and left halves of the housing manufactured separately and connected after assembly of the mechanism. The upper part of the housing has the screw rotating wheel, placed in a slot for the screw rotating wheel and the fixing button placed in the side slot of the housing. The truncated front part of the housing has recesses on its both sides for the operator's left thumb and left forefinger to hold the device fixed in position, wherein the recess for the left thumb also serves as a pad for fixing the coronary guide, and the rear upper part of the housing is made in the form of the support for the right hand having the shape of a tail. There is a rectangular window in the upper part of the housing made of clear material, under which the control ruler to determine and control the location of the mechanism fixing the coronary stent delivery system into the housing. The maximum distance of moving of the mechanism fixing the coronary stent delivery system in each side is limited with the ruler's size based on the size of the device. The front and rear parts of the housing have holes for insertion of the coronary stent delivery system into the housing.

There is the screw rotating wheel in the housing between the truncated front part and the cylindrical part; the upper part of said wheel bulges over the housing and the lower is inside the housing made as a single detail with a horizontal screw inside the housing placed on the runner and able to move along inside the housing, on which axis the coupling with the fixing element made of the elastic element at points, where it contacts the delivery system is placed with a bushing, which can rotate around its axis; there is a plug comprising the side element and the tapered element and a spring contacting the edge of the side element via a through hole in the runner and fixed on the runner's wall on the side opposite to the button, placed on the opposite side of the runner.

In this case the side element of the plug is placed in the side hole of the runner, on the side, where the fixing button is, forming a rectangular block with a rounded side surface on the side of the fixing button; it has a through hole made by the cut on one side and the cut at an angle on the other side, such cuts made in a way that allows end-to-end placement of the tapered element therein, which lower part is cut at a certain angle and the upper part forms a cone emerging from the hole in the runner on the side of the coupling and contacting them. Both plug components have holes used to move the coronary stent delivery system, when the device is working. The side part of the runner on the side opposite to the fixing button has a spring contacting the surface of the plug side component.

Such structure allows fixing the coronary stent delivery system rigidly at its progressive movement back and forth making it rotating freely around its axis.

The side part of the housing has a fixing button, the internal part of which is placed inside the housing and is a rectangular plate. The fixing button is centered with reinforcement plates inside the housing, which allows it moving freely towards and from the plug. The button is placed in such a way that when it is pushed its inner surface presses on the rounded surface of the side plug element, which in turn presses on the tapered plug, this leading to opening the coupling and the coronary stent delivery system undergoes lock release. Here, the cross-cut end of the plug side element placed on the side of the spring goes into the through hole in the runner and presses on the spring in the runner channel.

To return the fixing mechanism into the initial position with the closed coupling, the button is released, the spring opens pulling the plug side element into the initial position. Here, the tapered element also returns to its initial position due to elasticity of the coupling thereby fixing the coronary stent delivery system inside the device.

Thus, when the button is released the coupling clamping leads to the coronary stent delivery system being fixed, and when the button is pushed pressure is applied to the plug unclamping the coupling and making the coronary stent delivery system free to move along the axis.

In the preparation for use, a coronary stent carried by the delivery system is first progressively inserted inside the device, to perform this operation the operator presses fixing button, the inner rectangular part presses the plug, which makes the coupling to unclamp, and through the holes of the housing the delivery system of a coronary stent is placed. When no pressure is exerted on the fixing button the spring moves the side element of the plug, thus returning the tapered element and the button in the initial position, and the coupling clams fixing the delivery system. The operator manually advances the coronary stent carried by the delivery system placed within the device to the affected area of the coronary artery. Then, pressure is again exerted on the outer pressure surface of the release mechanism, the pressure roller causes the protrusions on the flaps to contract, the flaps open at the gripping point of the delivery system on the bushing side, and the operator brings the device to a necessary distance for fixing the same with his/her left arm, releases the pressure surface, and rotates the cylindrical rear part of the housing with his/her right arm to convert the rotary motion of the rear part of the housing into longitudinal translational motion of the runner with the gripping means along the guides inside the housing to displace the delivery system to a necessary distance for positioning of the coronary stent. Also when the operator pushes the button, he/she may return the mechanism fixing the coronary stent delivery system into the initial position by rotating the wheel in the required direction, which allows further positioning of the coronary stent forward or back within the range of the ruler in each side.

### Invention Embodiment Example 2.

The device consists of the housing with the tail and screw rotating wheel, screw placed thereon and made as a single element, the rotation gear contacting the screw and the pressure cylinder placed on the button with components for spring placement, which hold the delivery system guide.

The cylindrical housing is made from plastic using the casting method under pressure c pebbled at spots, where the operator touches the device, with the right and left halves of the housing manufactured separately and connected after assembly of the mechanism. The cylindrical rear part of the housing is connected to the truncated front part of the housing so as to be rotatable around its axis relative to the said front part. The upper part of the housing has the screw rotating wheel, placed in a slot for the screw rotating wheel and the fixing button placed in the side slot of the housing. The truncated front part of the housing has recesses on its both sides for the operator's left thumb and left forefinger to hold the device fixed in position, wherein the recess for the left thumb also serves as a pad for fixing the coronary guide, and the rear upper part of the housing is made in the form of the support for the right hand having the shape of a tail.

There is the screw rotating wheel in the housing between the truncated front part and the cylindrical part; the upper part of said wheel bulges over the housing and the lower is inside the housing made as a single detail with a horizontal screw inside the housing contacting the rotation gear placed on the pressure cylinder, which is able to rotate and contacting the fixing button element, on which springs the coronary stent delivery system guide is placed; the outer side of the system has a shape of a cylinder inside the housing.

In an embodiment, the device may be already provided with the delivery system fixed therein. In this case, the stage of inserting in advance the coronary stent carried by the delivery system inside the device is omitted. The coronary stent carried by the delivery system is manually advanced to the affected area of the coronary artery and then pressure is exerted on the pressure surface, the flaps unfold at the gripping point of the delivery system, and the operator brings the device to a necessary distance for fixing the same with his/her left arm, releases the pressure surface, and rotates the cylindrical rear part of the housing with his/her right arm to displace progressively the delivery system to a necessary distance for positioning the coronary stent within the artery. When pressure is exerted on the pressure surface, by fixing the delivery system, the operator may also return to initial position the mechanism fixing the coronary stent delivery system by rotating the cylindrical portion of the housing in the necessary direction so that further positioning of the coronary stent forwards and backwards may be continued within the dimensions of the guides inside the housing in each of the directions.

In an embodiment, for a safe placing of the stent on the delivery system, a hollow tube prearranged inside the device may be used for inserting therein the delivery system carrying the stent following which the tube is removed from the device.

Listed below are the attached drawings wherein:

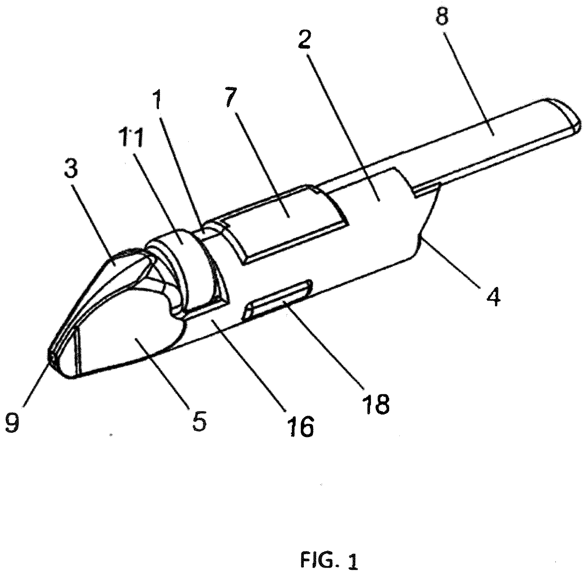

Fig. 1 is a general view of the device, showing:
   1 - device housing,
   2 - cylindrical portion of the front part of the housing,
   3 - truncated front part of the housing,
   4 - back portion of the housing,
   5 - recess for left arm fingers,
   7 - clear window,
   8 - right hand support in the form of the tail,
   9 - holes in the housing for the delivery system positioning,
   11 - screw rotating wheel,
   18 - fixing button.
Fig. 2 is a top view showing:
   1 - device housing,
   2 - cylindrical portion of the housing,
   3 - truncated front part of the housing,
   5 - recess for left arm fingers,
   7 - clear window,
   8 - right hand support in the form of the tail,
   11 - screw rotating wheel,
   18 - fixing button.
Fig. 3 is the side view, from the left side, from the side of the fixing button, showing:
   1 - device housing,
   2 - cylindrical portion of the front part of the housing,
   3 - truncated front part of the housing,
   4 - back portion of the housing,
   5 - recess for left arm fingers,
   8 - right hand support in the form of the tail,
   11 - screw rotating wheel,
   18 - fixing button
Fig. 4 is the cross-sectional general view of the device, showing:
   1 - device housing,
   6 - runner's position scale,
   8 - right hand support in the form of the tail,
   9 - holes in the housing for the delivery system positioning,
   10 - delivery system,
   11 - screw rotating wheel,
   12 - screw,
   13 - runner,
   15 - thread element for screw passage,
   17 - runner arrow,
   18 - fixing button,
   19 - guide buttons,
   20 - coupling,
   29 - housing halves fixing elements.

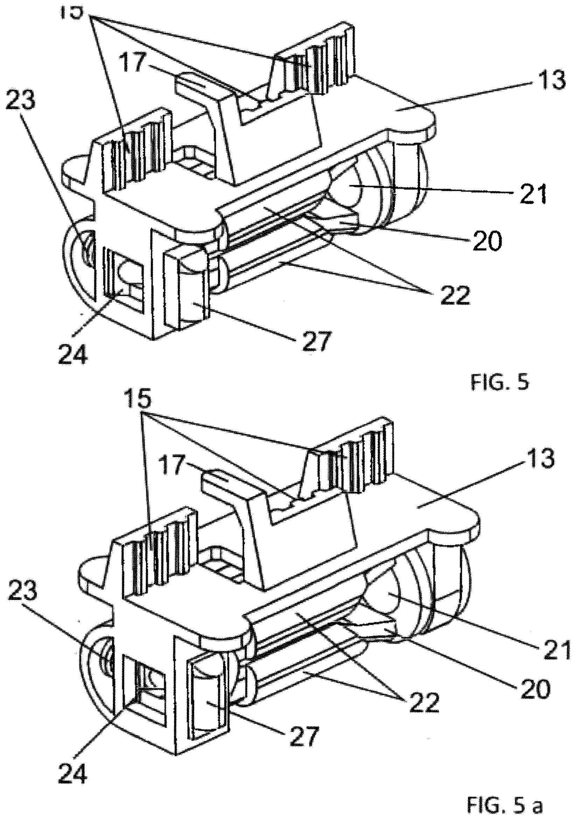

Fig. 5 is the general view of the device assembly with open coupling included, showing:
   13 - runner,
   15 - thread element for screw passage,
   17 - runner arrow,
   20 - coupling,
   21 - hole in a coupling for the delivery system,
   22 - fixing element,
   23 - plug spring,
   24 - tapered plug,
   27 - pressure plug.
Fig. 5a is the general view of the device assembly with closed coupling included, showing:
   13 - runner,
   15 - thread element for screw passage,
   17 - runner arrow,
   20 - coupling,
   21 - hole in a coupling for the delivery system,
   22 - fixing element,
   23 - plug spring,
   24 - tapered plug,
   27 - pressure plug.

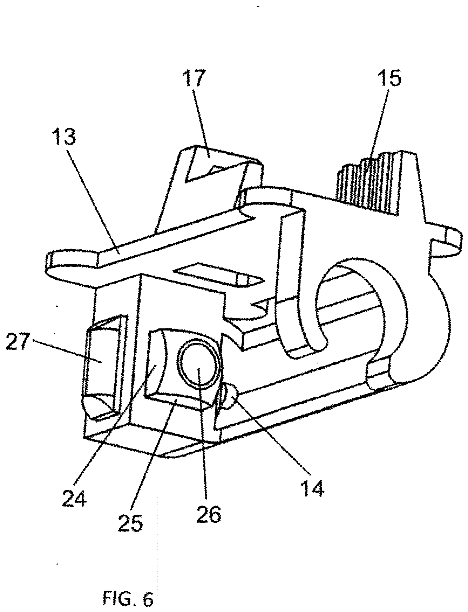

Fig. 6 is the general view of a runner, showing:
   13 - runner,
   14 - channel in a runner for a spring,
   15 - thread element for screw passage,
   17 - runner arrow,
   24 - tapered plug,
   25 - expanding surface of the tapered plug,
   26 - hole in the tapered plug element for positioning of the delivery system,
   27 - pressure plug.

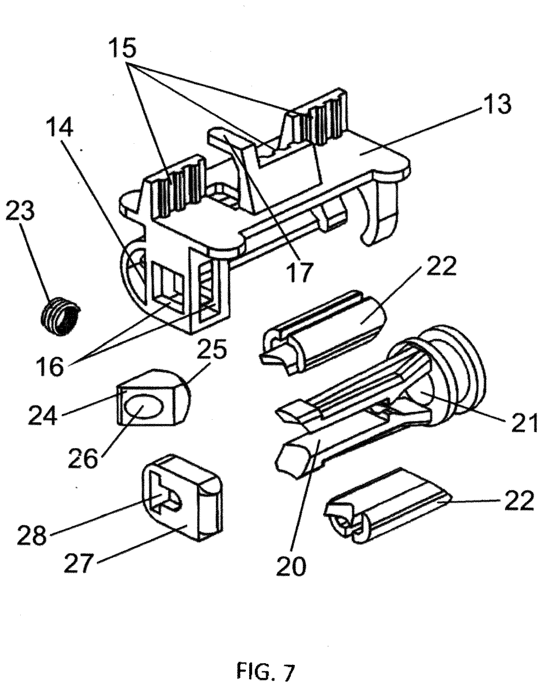

Fig. 7 is the general view of the disassembled delivery mechanism, showing:
   13 - runner,
   14 - channel in a runner for a spring,
   15 - thread element for screw passage,
   16 - hole in the runner to position plugs,
   17 - runner arrow,
   20 - coupling,
   21 - hole in a coupling for the delivery system,
   22 - fixing element,
   23 - plug spring,
   24 - tapered plug,
   25 - expanding surface of the tapered plug,
   26 - hole in the tapered plug element for positioning of the delivery system,
   27 - pressure plug.
   28 - hole in the pressure plug for positioning of the tapered plug.

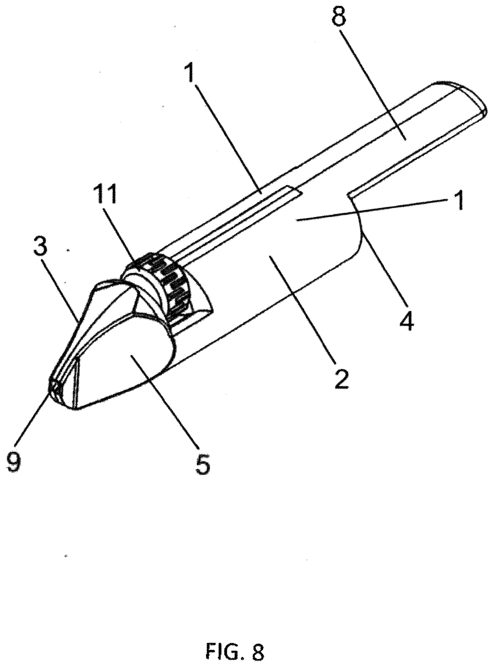

Fig. 8 is the general view, showing:
   1 - device housing,
   2 - cylindrical portion of the housing,
   3 - truncated front part of the housing,
   4 - back portion of the housing,
   5 - recess for left arm fingers,
   8 - right hand support in the form of the tail,
   11 - screw rotating wheel.

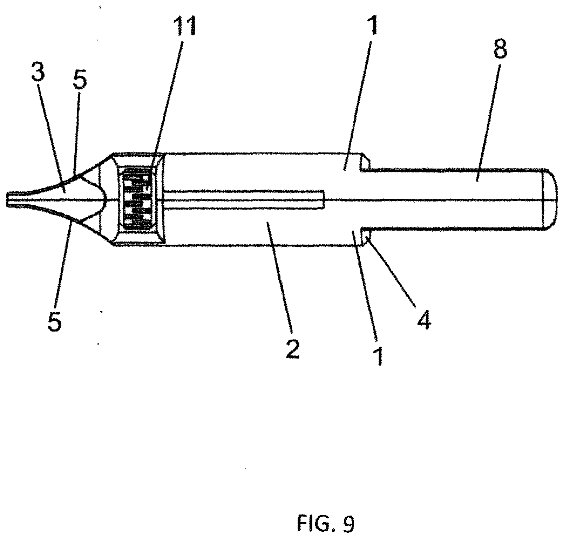

Fig. 9 is the top view, showing:
   1 - device housing,
   2 - cylindrical portion of the housing,
   3 - truncated front part of the housing,
   4 - back portion of the housing,
   5 - recess for left arm fingers,
   8 - right hand support in the form of the tail,
   11 - screw rotating wheel.

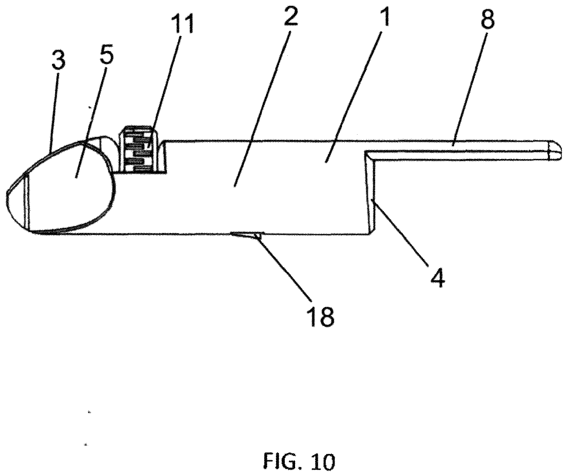

Fig. 10 is the side view, showing:
   1 - device housing,
   2 - cylindrical portion of the housing,
   3 - truncated front part of the housing,
   4 - back portion of the housing,
   5 - recess for left arm fingers,
   8 - right hand support in the form of the tail,
   11 - screw rotating wheel,
   18 - button.

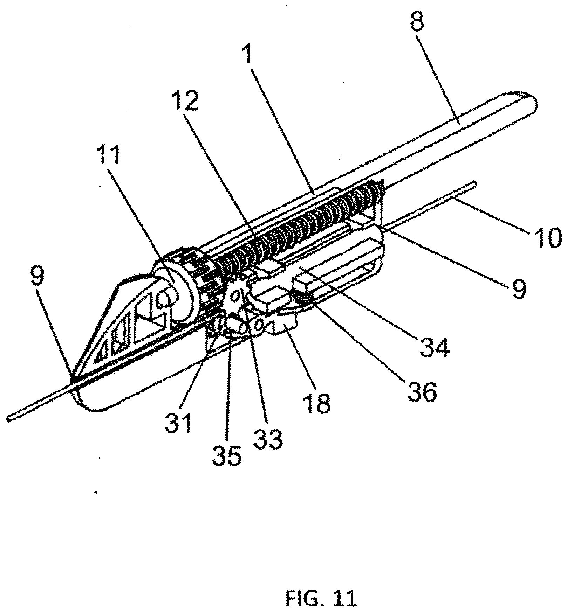

Fig. 11 is the cross-sectional view of the device, showing:
   1 - device housing,
   8 - right hand support in the form of the tail,
   9 - holes in the housing for the delivery system positioning,
   10 - delivery system,
   11 - screw rotating wheel,
   12 - screw,
   18 - button,
   31 - hold-down cylinder,
   33 - rotation gear,
   34 - guides for the delivery system inside the housing,
   35 - place of button connection to the hold-down cylinder,
   36 - spring.

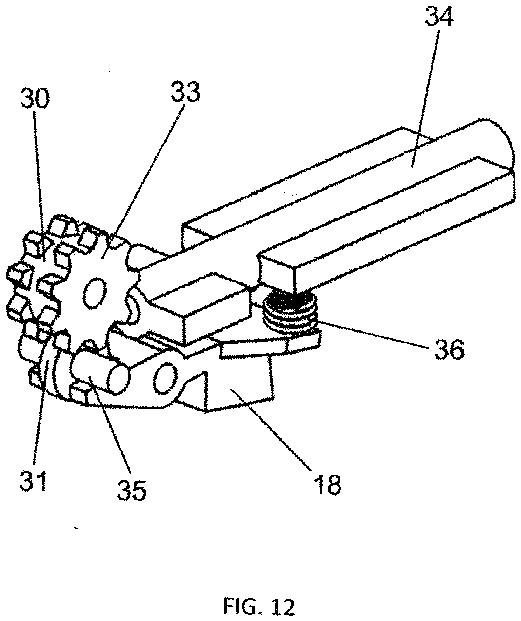

Fig. 12 is the general view of the fixing mechanism, showing:
   18 - button,
   30 - area of the delivery system adhesion to the rotation gear,
   31 - hold-down cylinder,
   33 - rotation gear,
   34 - guides for the delivery system inside the housing,
   35 - place of button connection to the hold-down cylinder,
   36 - spring.

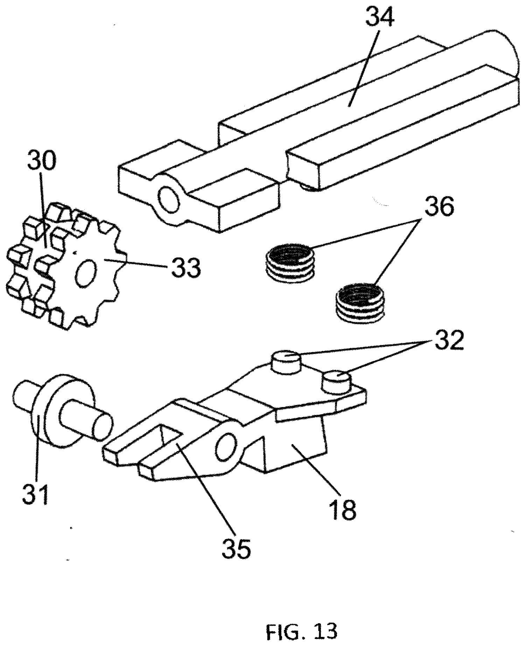

Fig. 13 is the view of the disassembled fixing mechanism, showing:
   button - 18,
   30 - area of the delivery system adhesion to the rotation gear,
   31 - hold-down cylinder,
   33 - rotation gear,
   34 - guides for the delivery system inside the housing,
   35 - place of button connection to the hold-down cylinder,
   36 - spring.

The invention will be described below with reference to the attached drawings.

### Invention embodiment, example 1

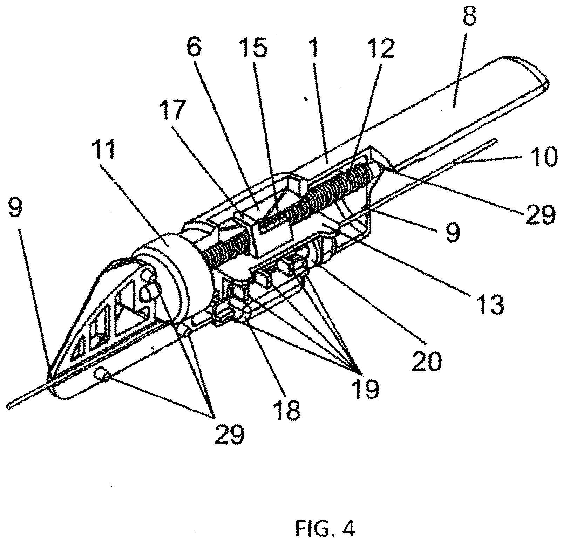

The device comprises a housing 1 (Fig. 1) formed of plastic by injection molding, wherein its surface is pebbled where it contacts the operator's arm 5 (Fig. 1, Fig. 2, Fig. 3), and the housing left and right halves (Fig. 1) are **manufactured** separately and assembled after placing therein a screw 12 (Fig. 4) with the rotation wheel 11 (Fig. 1, Fig. 2, Fig. 3, Fig. 4), with a runner 13 (Fig. 4, Fig. 5a, Fig. 5, Fig. 6), a clear window 7 (Fig. 1, Fig. 2, Fig. 3), thread elements 15 (Fig. 5, Fig. 5a, Fig. 6, Fig. 7), runner arrow 17 (Fig. 4. Fig. 5, Fig. 5a, Fig. 6, Fig. 7), elastic coupling 20 (Fig. 4, Fig. 5, Fig. 5a, Fig. 7), with elastic fixing elements placed thereon 22 (Fig. 5, Fig. 5a, Fig. 6, Fig. 7), tapered plug 24 (Fig. 5, Fig. 5a, Fig.7) and pressure plug 27 (Fig. 6, Fig. 5a, Fig. 7), plug spring 23 (Fig. 5, Fig. 5a, Fig. 7), placed in the channel 14 (Fig. 5, Fig. 5a, Fig. 7) of the runner 13 (Fig. 5, Fig. 5a, Fig. 7).

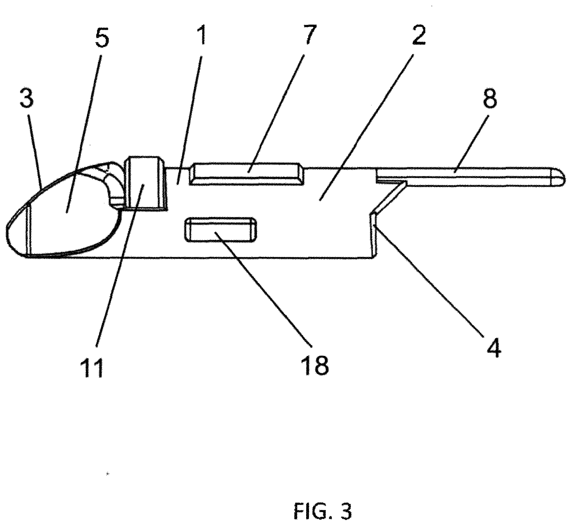

The housing consists of the front truncated part 3 (Fig. 1) with a through hole 9 (Fig. 1) for the delivery system 10 (Fig. 4) and a recess for left arm fingers 5 (Fig. 1), back part of the housing 4 (Fig. 1, Fig. 3) with a through hole for the delivery system 9 (Fig. 1, Fig. 4), above which a hand support is made 8 (Fig. 1, Fig. 4) in the form of a tail, and in a central cylindrical part 2 (Fig. 1). In the upper part of the housing 1 (Fig. 1) there is a clear window 7 with a control scale with guide marks (Fig. 1), on a runner 13 (Fig. 4, Fig. 5, Fig. 6), there is a fixing button on the left side of the housing 18 (Fig. 1). There is an upper part of the rotation wheel 11 (Fig. 1) of the screw 12 (Fig. 4) between the frontal truncated part 3 and the cylindrical part of the housing 2 (Fig. 1). The mechanism placed in the housing 1 (Fig. 1) comprises the screw 12 (Fig. 4), on which the screw rotating wheel 11 (Fig. 4) is firmly fixed and bulging from the outer side of the housing 1 (Fig. 1), and there is a runner 13 (Fig. 4) on the opposite side, with elastic coupling **20** (Fig. 4, Fig. 7) placed on its axis.

In the inner part of the housing, there is an internal fixing element 18 (Fig. 3, Fig. 4) in the hole for the button with guides 19 (Fig. 4), which may be moved both to the tapered plug 24 (Fig. 5, Fig. 5a, Fig. 6), comprising the tapered element 23 and pressure plug 27 (Fig. 5, Fig. 5a, Fig. 6, Fig. 7), and from it in order to open or close the coupling 20 (Fig. 5, Fig. 5a, Fig. 7) as a result of clamping or declamping of the resin coupling 20, in case of placing the delivery system 10 inside the device.

There is the hole 21 (Fig. 5, Fig. 5a, Fig. 7) of the runner 13 (Fig. 5, Fig. 5a, Fig. 7) in coupling 20, with horizontal channel holes with a possibility of positioning of the delivery system 10 (Fig. 4) therein.

To position the delivery system 10 inside the device, the operator pushes the fixing button 18 (Fig. 1, Fig. 3), internal pressure plug 27 (Fig. 5, Fig. 5a), the tapered plug 24 (Fig. 5, Fig. 5a, Fig. 7), expanding surface of the tapered plug 25 (Fig. 7) pulls out the tapered plug 24 (Fig. 5, Fig. 5a, Fig. 7) towards the coupling with fixing elements placed thereon 22 (Fig. 5, Fig. 5a, Fig. 7), tapered plug 24 (Fig. 5, Fig. 5a, Fig. 7) declamps the coupling 20 (Fig. 5, Fig. 5a, Fig. 7), which produces in a free through hole for positioning of the delivery system 21 (Fig. 5, Fig. 5a) inside the device. The device is placed through holes 9 (Fig. 1, Fig. 4) in the frontal and back parts of the device 1 (Fig. 1) and the truncated front part of the housing 3 (Fig. 1) of the delivery system 10 (Fig. 4). After that the fixing button 18 is released (Fig. 5), spring 23 (Fig. 5), placed in channel 14 (Fig. 7) of the runner 13 (Fig. 7), pulls out the tapered plug 25 the expanding surface 24 and expanding surface 25 (Fig. 5), the tapered plug 24 (Fig. 5) takes the initial position as a result of clamping of the coupling 20 (Fig. 5).

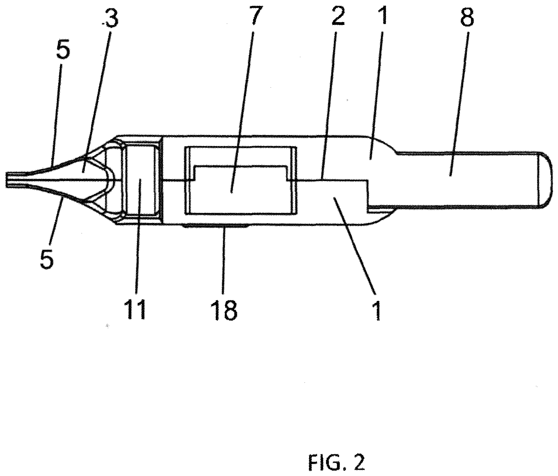

Using the screw rotating wheel 11 (Fig. 1) the operator, transforming the rotational moving of the wheel 11 (Fig. 4) into the progressive movement of the runner 13 (Fig. 4), moves the delivery system 10 (Fig. 4) horizontally inside the device with a possibility of its rotation around its axis controlling the movement via the window 7 (Fig. 1, Fig. 2) with the control scale 6 (Fig. 4) with risk profiles. The device is made in such a way that the runner 13 (Fig. 4) moves several millimeters forward per one rotation of the screw rotating wheel 11 (Fig. 4).

In an embodiment of the invention, the device may be used with a prior fixed delivery system 10 (Fig. 4), where the need in prior placement of the delivery system inside the device is excluded.

In another embodiment of the invention for safe positioning of the stent in the delivery system, an empty tube may be used, which has been prior placed inside the device, in which the delivery system 10 (Fig. 4) with the stent is inserted, then the tube is removed from the device.

### Device embodiment Example 2.

The device comprises a housing 1 (Fig. 8, Fig. 9) with a tail 8 (Fig. 8, Fig. 9, Fig. 10) and the screw rotating wheel 11 (Fig. 8, Fig. 9, Fig. 11) placed therein, screw 12 (Fig. 11), made as a single element, rotation gear 33 (Fig. 11, Fig. 12, Fig. 13), interacting with the screw 12 (Fig. 11), and hold-down cylinder 31 (Fig. 11, Fig. 12, Fig. 13), placed in the internal side of the button 18 (Fig. 11, Fig. 12, Fig. 13), having elements 32 (Fig. 13) for placement of springs 36 (Fig. 11, Fig. 12, Fig. 13), on which the guide for the delivery system 34 (Fig. 11, Fig. 12, Fig. 13) is placed.

Housing 1 (Fig. 8) is cylindrical, formed of plastic by injection molding, wherein its surface is pebbled where it contacts the operator's arm, and the housing left and right halves 1 (Fig. 8) are manufactured separately and connected after completion of the device assembly. In the upper part the housing 1 (Fig. 8) is equipped with the screw rotating wheel 11 (Fig. 8, Fig. 9, Fig. 10), placed in the hole for the screw rotating wheel and the fixing button 18 (Fig. 10, Fig. 11, Fig. 12, Fig. 13), placed in the side hole of the housing. The frontal tapered part of the housing fitted for holding it with the thumb and index of the operator's left arm have recesses (Fig. 8, Fig. 9, Fig. 10), allowing the operator to fix the device in a certain position, where the recess for the thumb of the left arm also serve as a ground for coronary conductor fixation, and the back part of the housing 1 is made as the right hand support in the form of the tail 8 (Fig. 8, Fig. 9, Fig. 10) .

There is the screw rotating wheel 11 (Fig. 8, Fig. 9, Fig. 10) in the housing between its truncated part 3 (Fig. 8, Fig. 9, Fig. 10) and the cylindrical part 2 (Fig. 8, Fig. 9, Fig. 10), its upper part bulges over the housing, and the lower is inside the housing as a single detail with a horizontal screw 12 (Fig. 11) inside the housing 1 (Fig. 11), which interacts with the rotation gear 33 (Fig. 11), placed on the pressure plug 31 (Fig. 11, Fig. 12, Fig. 13) which may rotate and interacting with the element of the fixing button 35 (Fig. 13), on which springs 36 (Fig. 11, Fig. 12, Fig. 13) the guide of the coronary stent delivery system 34 (Fig. 11, Fig. 12, Fig. 13) is placed, its outer part is a cylinder inside the housing.

For placing a delivery system 10 (Fig. 11) inside the device, the operator exerts pressure with his/her right arm fingers on the pressure surface 18 (Fig. 11, Fig. 12, Fig. 13), the internal element 35 (Fig. 12) is placed down and removes pressure between 33 (Fig. 12) and the hold-down cylinder 31 (Fig. 12), with formation of a through hole 9 (Fig. 11) for insertion of the delivery system 10 (Fig. 12) to the guides 34 (Fig. 12) inside housing 1 through holes 9 (Fig. 11), then the fixing button 18 (Fig. 11, Fig. 12, Fig. 13) goes down.

Using holes 9 (Fig. 8) in the front and back sides of the housing 1 (Fig. 1) and the truncated front part 3 (Fig 11) the delivery system 10 (Fig. 11) is inserted to the device. After that the fixing button 18 is released (Fig. 11), and the delivery system 10 (Fig. 11) is fixed inside the device. Using the screw rotating wheel 11 (Fig. 11) the operator, transforming the rotational moving of the wheel 11 (Fig. 11 into the progressive movement of the runner 12 (Fig. 11) and rotational movement of the rotation gear 33 (Fig. 11), moves the delivery system 10 (Fig. 11) horizontally inside the device

Another embodiment of the invention has the delivery system 10 (Fig. 4) prior fixed therein, where the need in prior placement of the delivery system inside the device is excluded.

Another embodiment of the invention for safe positioning of the stent in the delivery system, an empty tube may be used, which has been prior placed inside the device, in which the delivery system 10 (Fig. 4) with the stent is inserted, and then the tube is removed from the device.

## Claims

1. A device for positioning of a coronary stent within coronary arteries, **characterized in that** it comprises a housing with the screw rotating wheel, which upper part bulges over the housing and the lower part is inside the housing made as a single element with the screw placed in thread elements of the runner with a possibility of its horizontal movement; there is elastic coupling placed on the runner's axis with elastic fixing elements tapered in the front part with recesses for the operator's arm and the upper lateral side in the form of a tail as the right hand support, with the window and a scale for the runner's position determination in the upper part; there are through holes in the frontal and the back parts of the housing for placement of the delivery system with the coronary stent, having a fixing button in it left side, its internal part is placed inside the housing and is a rectangular plate, centering reinforcement plates inside the housing, which allow it moving freely towards the plug, placed on the opposite side of the runner and the tapered plug, the pressure plug is placed in the side hole of the runner on the side of the fixing button and has a cut inside and a cut on the side opposite to the fixing button, where the pressure plug is placed end-to-end the tapered plug at the cut point, the lower edge part of which is also cut at certain angle, and the upper opposite part is a tapered plug emerging from the hole in the runner from the side of the coupling, touching it; there are holes in the pressure and tapered plugs, in which the delivery system of the coronary stent is moved at the open coupling placed on the runner, a spring connected via the side hole in the runner with the side element edge at the cut point is fixed on the side opposite to the fixing button.

2. The device, according to example 1, is **characterized in that** the housing is formed of plastic, wherein its surface contacting the operator's arm is pebbled.

3. The device, according to example 1, is **characterized in that** the delivery system with the stent is prior fixed therein.

4. The device, according to example 1, is **characterized in that** an empty tube is prior inserted therein for placement of the delivery system with the stent.

5. A device for positioning of a coronary stent within coronary arteries is **characterized in that** it comprises a housing with the screw rotating wheel, which upper part bulges over the housing and the lower part is inside the housing made as a single element with the screw placed interacting with the rotation gear placed on the pressure plug with a possibility of rotating, placed on the internal element of the fixing button having springs for placement of the guide for the delivery system.

6. The device, according to example 5, is **characterized in that** the housing is formed of plastic, wherein its surface contacting the operator's arm is pebbled.

7. The device, according to example 5, is **characterized in that** the delivery system with the stent is prior fixed therein.

8. The device, according to example 5, is **characterized in that** an empty tube is prior inserted therein for placement of the delivery system with the stent.

9. A method for positioning of a coronary stent within coronary arteries, **characterized in that** when a coronary stent delivery system is placed within the device housing the coronary stent is gradually inserted with the delivery system inside the device, to do which the operator presses on the fixing button, the internal rectangular part of the button presses on the plug, declamping the coupling and the coronary stent delivery system is placed via the through holes in the housing. After stopping pressing the fixing button, the spring pulls out the side element of the plug thereby returning the tapered element and the button into the initial position, and the coupling declamps holding the delivery system, and then the operator manually passes the coronary stent through the delivery system placed in the device, till the area of the coronary artery lesions has been reached, then the operator pushes the fixing button again, the coupling opens and the operator moves the device at the distance necessary for its fixing with the left arm's fingers, releases the fixing button and rotates the screw rotating wheel using his/her right hand transforming the rotational movement of the rotating wheel into the progressive movement of the runner and moves the delivery system at the distance required for positioning of the coronary stent inside the vessel, pressing the button the operator may also return the mechanism fixing the coronary stent delivery system into the initial position by rotating the wheel in necessary direction, allowing to continue further positioning of the coronary stent back and forth within the range of the scale in any direction.

10. A method for positioning of a coronary stent within coronary arteries, **characterized in that** when a coronary stent delivery system is placed within the device housing, the fixing button placed in the side part of the housing is pushed, its internal element goes down, removing pressure between the rotation gear and the hold-down cylinder and forming the through hole for placement of the delivery system in the guides through the front and the back parts of the housing, then the fixing button is released, the delivery system is inserted, the fixing button is released fixing the delivery system inside the device, using the screw rotating wheel the operator transforming the rotational movement of the rotating wheel into the progressive movement of the screw and rotational movement of the rotation gear, moves the fixed delivery system horizontally inside the device at the required distance.
